**Europäisches Patentamt**

**European Patent Office**

**Office européen des brevets**

(19)

(11) Veröffentlichungsnummer: **0 065 716**

**A2**

(12) # EUROPÄISCHE PATENTANMELDUNG

(21) Anmeldenummer: 82104201.7

(22) Anmeldetag: 13.05.82

(51) Int. Cl.³: **A 61 F 7/10**

(30) Priorität: 21.05.81 DE 8115207 U

(43) Veröffentlichungstag der Anmeldung:
01.12.82 Patentblatt 82/48

(84) Benannte Vertragsstaaten:
AT BE CH DE FR GB IT LI LU NL SE

(71) Anmelder: Hospipharm AG
Ergerta 271
Balzers(LI)

(72) Erfinder: Hecht, Manfred
Gerresheimer-Land-Strasse 110
D-4000 Düsseldorf 12(DE)

(74) Vertreter: Ruschke, Hans Edvard et al,
Patentanwälte Dipl. Ing. Olaf Ruschke Dipl. Ing. Hans E.
Ruschke Dipl.-Ing. Jürgen Rost Dipl.-Chem Dr. U. Rotter
Pienzenauerstrasse 2
D-8000 München 80(DE)

(54) Zur therapeutischen Behandlung der Kopfhaut mit Kälte vorgesehene flexible Kompresse.

(57) Zur therapeutischen Behandlung der Kopfhaut mit Kälte vorgesehene flexible Kompresse, die in flach ausgebreitetem Zustand im wesentlichen eben ist, bestehend aus mehreren, von einer Kunststoffolie umgrenzten, segmentartigen flachen Kammern, die mit einer gelartigen Masse gefüllt sind und in thermischen Kontakt mit der Kopfhaut dieser Wärme entziehen. Die Erfindung ist darin zu sehen, daß ein kreisrundes, zentrales Schädeldeckensegment (A) vorgesehen ist, das längs seines gesamten Umfanges an vier etwa gleichgroßen Bogenabschnitten (1, 2, 3, 4) mittels einer den Umfang bildenden Schweißnaht mit vier lappenartigen Segmenten (B, B', C und D) verbunden ist, die sich in um 90° gegeneinander versetzten Richtungen (N, S, O, W) nach auswärts von dem Segment (A) erstrecken und deren untereinander verbundenen, als Schweißnähte ausgeführten seitlichen Ränder zunächst in etwa radial nach auswärts und an schließend bis zum Ende der lappenartigen Segmente stärker in den Richtungen (N, S, O, W) verlaufen.

Fig. 3

EP 0 065 716 A2

- 3 -

---------------------------------------------------------------------

Zur therapeutischen Behandlung der Kopfhaut mit Kälte vorgesehene flexible Kompresse

---------------------------------------------------------------------

Die vorliegende Erfindung betrifft eine zur therapeutischen
Behandlung der Kopfhaut mit Kälte vorgesehene flexible Kompresse nach dem Oberbegriff des Schutzanspruchs 1.

Bei bestimmten Krebsformen hat eine medikamentöse Therapie
gute Erfolge gezeigt. Die hierfür geeigneten Medikamente
werden häufig intravenös verabreicht, haben jedoch die
unangenehme Nebenwirkung, daß sie auch zum totalen Haarausfall führen weil sie die Haarwurze-Zellenn schädigen. Um
diesen unangenehmen Nebeneffekt möglichst gering zu halten,
hat man sich mit gutem Erfolg darum bemüht, insbesondere
während der Phasen höchster Konzentration des Medikamentes
im Blut die Blutzufuhr zu den Haarwurzeln weitgehend zu

- 4 -

reduzieren, um damit die angesprochene Schädigung gering zu
halten. Dies kann dadurch bewirkt werden, daß die Kopfhaut
unterkühlt wird, wodurch sich die blutführenden Gefäße stark
zusammenziehen. Die Zellteilungsgeschwindigkeit wird durch
Kälte stark herabgesetzt. Das Medikament kann nur im Moment
der Zellteilung in die Zelle eindringen.Eine derartige
Abkühlung wird etwa durch im Handel befindliche Kaltkompressen bewirkt.

Es ist bereits eine Kompresse dieser Art bekannt, die unter
dem Warenzeichen "KOLD KAP" anderem von der Firma Hospipharm
GmbH in Deutschland vertrieben wird. Diese bekannte Kompresse besteht im wesentlichen aus zwei aktiven Teilen, nämlich
einem um den Kopf des Patienten herumzulegenden breiten
Band, mit dessen Mittelabschnitt die Grundfläche eines in
etwa gleichseitigen dreieckigen Segmentes verbunden ist. Die
bekannte Kompresse wird mit Klebebändern oder Bandagen auf
dem Kopf des Patienten befestigt. In dem genannten Band und
dem etwa dreieckigen Segment sind Kammern mit zwei Substanz
vorgesehen, die durch Druck zu einer endothermen Reaktion
veranlaßt werden, um der Kopfhaut des Patienten in der
beabsichtigten Weise Wärme zu entziehen.

Diese bekannte Kompresse hat mehrere Nachteile. Zum einen
ist die Anbringung der Kompresse nicht immer ohne Mithilfe

- 5 -

des Patienten möglich, weil sie während der Befestigung
leicht verrutscht. Zum anderen ist diese Kompresse nur
einmal verwendbar, und zwar wegen der aktiven Substanz, die
nach der geschilderten endothermischen Reaktion nicht wieder
aktivierbar ist. Außerdem liegt die damit erreichbare Temperatur (+ 24$^{\circ}$ C) nicht allzu niedrig, und schließlich ist
wegen der Geometrie der Kompresse nicht immer eine gleichmäßige Abkühlung erzielbar.

Hiernach wird es als die der vorliegenden Erfindung zugrundeliegende Aufgabe angesehen, eine leicht handhabbare, wiederverwendbare Kompresse vorzusehen, die eine gleichmäßige,
gute Abkühlung der Kopfhaut ermöglicht.

Diese Aufgabe wird erfindungsgemäß durch die Merkmale des
Schutzanspruches 1 gelöst. Da die Kompresse ein zentrales
kreisförmiges Schädeldeckensegment mit weiteren vier davon
ausgehenden lappenartigen Segmenten aufweist, ist eine gute
Anpassung an jede Kopfform möglich. Auch wird sie nach dem
Aufsetzen auf den Kopf des Patienten nicht verruschten, weil
sofort eine im wesentlichen gleichmäßige Gewichtsverteilung
gegeben ist. Die Abmessungen der einzelnen Segmente sind
dem Verlauf der wichtigsten blutführenden Gefäße angepaßt,
insbesondere im Nacken- und Schlägenbereich. Außerdem braucht
die Kompresse nicht nach Gebrauch weggeworfen zu werden, weil sie nach einer ausreichend langen erneuten Abkühlung in einem Kühlfach oder dergl. wieder zum Einsatz kommen kann.

Die in der Kompresse verwendete Gelmasse kann als thermoträge bezeichnet werden und beruht auf einer Glyzerinbasis mit Stärke als Bindemittel zur Eindickung, wobei ein Bakterizid als Zusatz vorgesehen ist.

Ein Ausführungsbeispiel der vorliegenden Erfindung wird im nachfolgenden unter Bezugnahme auf die beigefügten Zeichnungen beschrieben. In diesen zeigt

Fig. 1 eine Kompresse in Draufsicht, und zwar in flach ausgebreiteter Anordnung,

Fig. 2 einen Schnitt längs Linie I-I in Fig. 1, und

Fig. 3 die Kompresse nach dem Aufsetzen auf den Kopf eines Patienten.

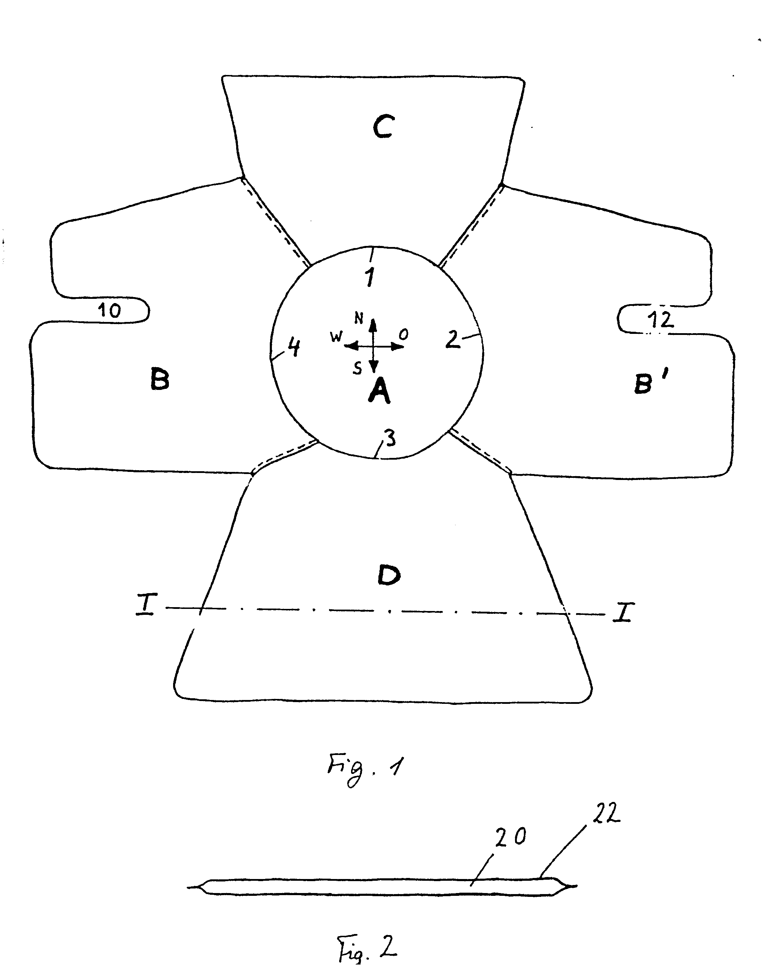

Die Kompresse besteht aus zwei übereinander angeordneten Kunststoffolien mit den aus Fig. 1 ersichtlichen Umrissen, wobei diese Kunststoffolien entlang der durchgezogenen schwarzen Linien miteinander verschweißt sind. Der dadurch entstehende Hohlraum in den einzelnen Segmenten A, B, B', C
und D ist mit einer Gel-Substanz auf Glyzerinbasis gefüllt,
die gut wärme- bzw. kältespeicherfähig ist. Das mit A bezeichnete kreisförmige Schädelsegment ist mit den anderen
lappenförmigen Segmenten B, B', C und D im Bereich der
kreisförmigen Schweißnaht verbunden, während die lappenförmigen Segmente auch untereinander längs der geraden
Schweißnähte verbunden sind, wie durch die gestrichelten
Linien angedeutet sein soll. B und B' bezeichnen die beiden
Seitensegmente, während C das Stirnsegment ist und D das
Hinterkopf- und Nackensegment bezeichnet.

Die untereinander verbundenen lappenförmigen Segmente B, B',
C und D sind mit dem kreisförmigen Schädeldeckensegment A
längs dessen Umfang verbunden, und zwar im Bereich von vier
etwa gleich großen Bogenabschnitten 1, 2, 3 und 4. Die
seitlichen Ränder der lappenförmigen Segmente verlaufen vom
kreisförmigen Umfang des Segmentes A ausgehend zunächst etwa
in radialer Richtung, um danach einen Richtungswechsel
vorzunehmen, d.h. stärker in den um 90° gegeneinander
versetzten Richtungen N, O, S und W zu verlaufen. Das Hinter-
kopf- und Nackensegment D ist um einiges länger als das
Stirnsegment C. Die beiden Seiten- und Ohrensegmente B, B'
weisen Schlitze 10, 12 für die Ohren des Patienten auf.
Diese Schlitze gehen vom äußeren oder (in Gebrauchslage) unteren Rand der Segmente B, B' aus und erstrecken sich geradlinig in Richtung auf das Segment A, und zwar etwa bis zur Hälfte der Länge der Segmente B, B'. Die genauen Maße einer Ausführungsform dieser Kompresse ergeben sich auf einer mit Maßen versehenen Kopie der Fig. 1, wobei allerdings darauf hinzuweisen ist, daß die Neuerung nicht auf diese Maße beschränkt ist, da je nach durchschnittlicher Kopfform, z.B. bei Kindern oder Erwachsenen, unterschiedliche Abmessungen notwendig sein können. Allerdings wird in der grundsätzlichen Formgebung der Kompresse ein wesentlicher der Neuerung gesehen.

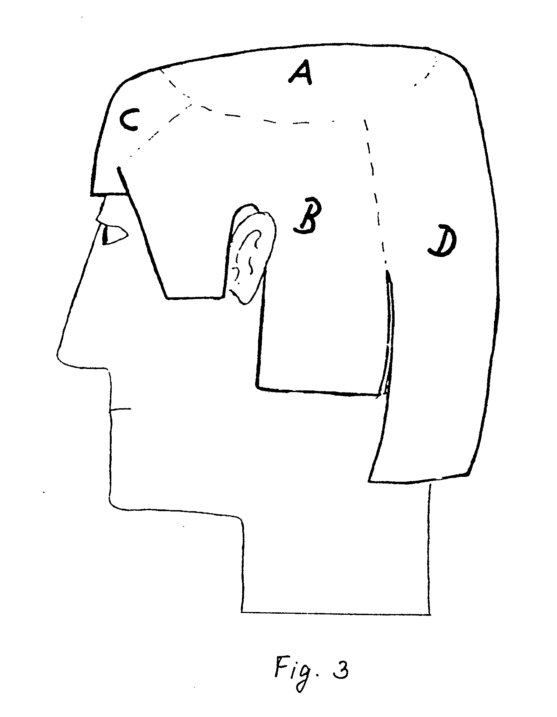

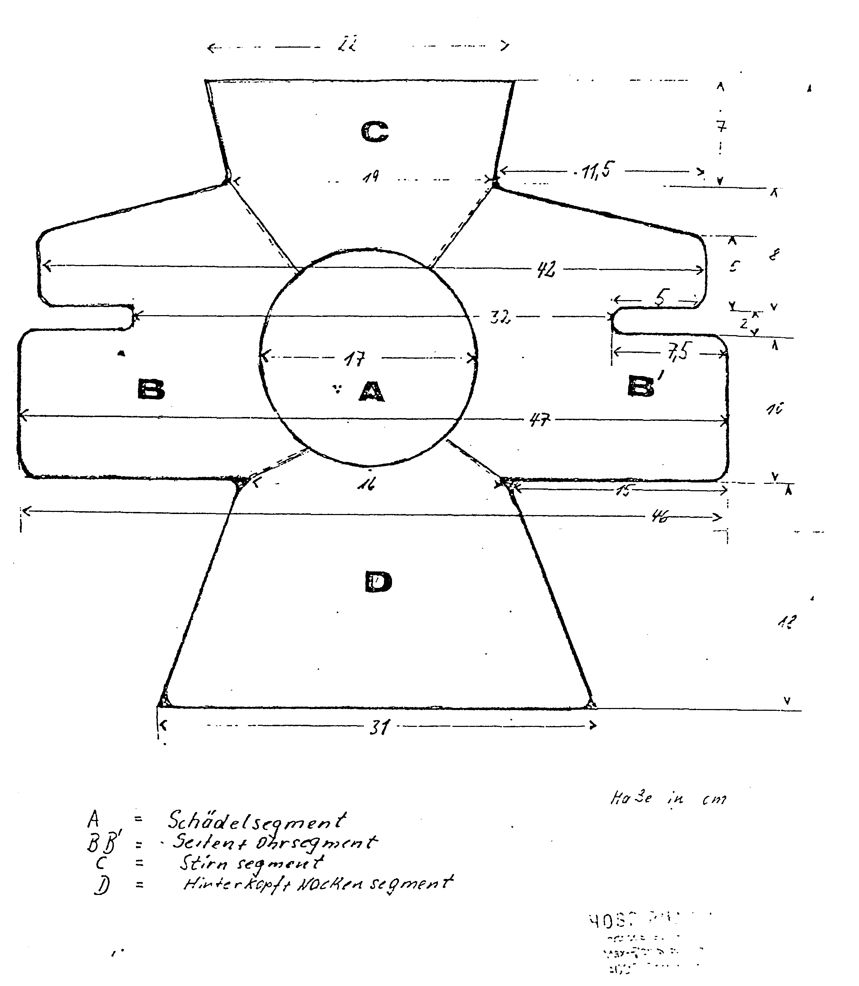

Wie schon angedeutet, zeigt Fig. 3 die Kompresse in der konkreten Anwendung auf dem Kopf eines Patienten. Fig. 2 zeigt einen Schnitt längs Linie I-I in Fig. 1 und soll die dreidrimensionale Formgebung der Kompresse verdeutlichen. Die die Abkühlung bewirkende Gelmasse ist in dem Hohlraum 20 des Segmentes D ebenso wie in den anderen Segmenten untergebracht. Die an den Rändern zusammengeschweißte Kunststoffolie 22 kann etwa aus eienr Polyäthylen-Folie mit einer Stärke von 100 µm bestehen. Diese Folie behält insbesondere auch bei den hier anzuwendenden niedrigen Temperaturen eine ausreichende Flexibilität.

**M U N C H E N**
Pienzenauerstr. 2
8000 München 80
Telefon: (089) 98 03 24,
98 72 58, 98 98 00
Kabel: Quadratur München
Telex: 5 227 67

**B E R L I N**
Kurfürstendamm 182/183
1000 Berlin 15
Telefon: (030) 8 83 70 711 / 79
Kabel: Quadratur Berlin

## RUSCHKE & PARTNER
## PATENTANWÄLTE

-1-

Dr.-Ing. Hans Ruschke* – bis 1980 –
Dipl.-Ing. Hans **0065716**
Dipl.-Ing. Olaf Ruschke
Dipl-Ing. Jürgen Rost
Dipl.-Chem. Dr. Ulrich Rotter

Zugelassen beim Europäischen Patentamt
Admitted to the European Patent Office

* In Berlin

HOSPIPHARM AG,

Ergerta 271, Balzers   (Liechtenstein)

-------------------------------------------------------------------------


S c h u t z a n s p r ü c h e


1. Zur therapeutischen Behandlung der Kopfhaut mit Kälte
vorgesehene flexible Kompresse, die in flach ausgebreitetem
Zustand im wesentlichen eben ist, bestehend aus mehreren,
von einer Kunststoffolie umgrenzten, segmentartigen flachen
Kammern, die mit einer gelartigen Masse gefüllt sind und in
thermischem Kontakt mit der Kopfhaut dieser Wärme entziehen,
dadurch gekennzeichnet, daß ein kreisrundes, zentrales
Schädeldeckensegment (A) vorgesehen ist, das längs seines
gesamten Umfanges an vier etwa gleichgroßen Bogenabschnitten
(1, 2, 3,4) mittels einer den Umfang bildenden Schweißnaht
mit vier lappenartigen Segmenten (B, B', C und D) verbunden ist, die sich in um 90 ° gegeneinander versetzte Richtungen (N, S, O, W) nach auswärts von dem Segment (A) erstrecken und deren untereinander verbundenen, als Schweißnähte ausgeführten seitlichen Ränder zunächst in etwa radial nach auswärts und anschließend bis zum Ende der lappenartigen Segmente stärker in den Richtungen (N, S, O, W) verlaufen.

2. Kompresse nach Anspruch 1, dadurch gekennzeichnet, daß die beiden einander gegenüberliegenden Seiten- und Ohrensegmente B, B' je mit einem etwa radial verlaufenden Schlitz (10, 12) zur Aufnahme der Ohren des Patienten versehen sind.

3. Kompresse nach Anspruch 1 oder 2, dadurch gekennzeichnet, daß das Hinterkopf- und Nackensegment D sich im wesentlichen weiter auswärts erstreckt als das gegenüberliegende Stirnsegment C.

0065716

1/3

Fig. 1

Fig. 2

Fig. 3

A = Schädelsegment
B B' = Seitent Ohrsegment
C = Stirn segment
D = Hinterkopft Nocken segment

Maße in cm